# EUROPÄISCHE PATENTSCHRIFT

(11) **EP 1 439 873 B1**
(45) Veröffentlichungstag und Bekanntmachung des Hinweises auf die Patenterteilung: **22.08.2007**
(21) Anmeldenummer: 02776590.8
(22) Anmeldetag: 30.10.2002
(51) Int. Cl.: A61M 5/50, A61M 5/28, A61M 5/32, A61M 5/34

(54) **EINWEG-INJEKTIONSVORRICHTUNG**
DISPOSABLE INJECTION DEVICE
DISPOSITIF D'INJECTION JETABLE

(30) Priorität: 31.10.2001 AT 17182001
(43) Veröffentlichungstag der Anmeldung: 28.07.2004
(73) Patentinhaber: Jütte, Werner, 1060 Wien (AT)
(72) Erfinder: Jütte, Werner, 1060 Wien (AT)
(86) Internationale Anmeldenummer: PCT/AT2002/000304
(87) Internationale Veröffentlichungsnummer: WO 2003/037410

(56) Entgegenhaltungen:
- WO-A-94/00172
- DE-C- 882 600
- US-A- 3 145 712
- US-A- 5 569 190
- US-A- 6 045 534

## Beschreibung

Die Erfindung betrifft eine Einweg-Injektionsvorrichtung mit einer Vorratskammer zur Aufnahme von Injektionsmedien und mit einem an der Vorratskammer vorgesehenen Injektionsausgang, der von einem Ende einer an beiden Enden zugeschärften Injektionsnadel durchdringbar ist, zur Durchführung von subkutanen und intrakutanen Injektionen. Als Injektionsmedien kommen beispielsweise Lösungen oder Suspensionen in Frage.

In der Prophylaxe, bei chronischen Erkrankungen oder bei Notfällen ist es wünschenswert, dass auch eine ungeschulte Hilfsperson oder der Patient selbst die erforderlichen Injektionen rasch, einfach, gefahrlos und möglichst mit einer Hand durchführen kann. Bekanntlich werden allerdings weltweit auch zahlreiche Menschen durch Wiederverwendung gebrauchter Spritzen oder durch Verletzung mit der Nadel der weggeworfenen Spritzen mit gefährlichen Krankheiten infiziert. Von großem Vorteil wäre es daher, eine Einweg-Injektionsvorrichtung zu schaffen, welche die genannten Aufgaben erfüllt und zusätzlich die Wiederverwendung der Vorrichtung unmöglich macht.

Für die Humanmedizin stand bisher keine Einweg-Injektionsvorrichtung zur Verfügung, die nicht unbedingt von medizinischen Fachkräften bedient werden muss, sondern auch von ungeschulten Personen oder vom Patienten selbst, insbesondere auch von einem sehbehinderten, in seiner psychischen oder geistigen Leistung eingeschränkten oder motorisch gestörten Patienten - sogar in Notfallsituationen - gefahrlos und mit nur einer Hand bedient werden kann. Die bekannten Spritzampullen stellen nach ihrer Verwendung zudem immer noch eine potentielle Gefahr für den Anwender selbst oder für andere Personen dar. Der gleiche Mangel besteht hinsichtlich der Verabreichung von intrakutanen Injektionen, etwa im Rahmen von Schutzimpfungen und Therapieprogrammen sowie in der Notfallmedizin.

In der US 4,955,871 A ist eine Einweg-Injektionsspritze vom eingangs definierten Typ beschrieben. Die bekannte Spritze weist eine übliche, entfernbare Nadelschutzkappe auf, sie ist keinesfalls mit einer Hand und selbst nach Einschulung nicht von jedermann bedienbar. An einer gebrauchten Spritze dieser Art kann man sich überdies immer noch verletzen.

Die US 5,569,190 A ist auf einen elektrisch betriebenen, hypodermalen Dispenser gerichtet, mit welchem Fluide aus einem Vorratsbehälter unter die Haut injiziert werden können. Der Dispenser muß insbesondere eine spezielle, wiederaufladbare Energiequelle enthalten. Das Gerät ist im Zusammenwirken mit Hochdruck-("jet"-) Injektoren - z. B. für den Einsatz bei Massenimpfungen - genauer beschrieben.

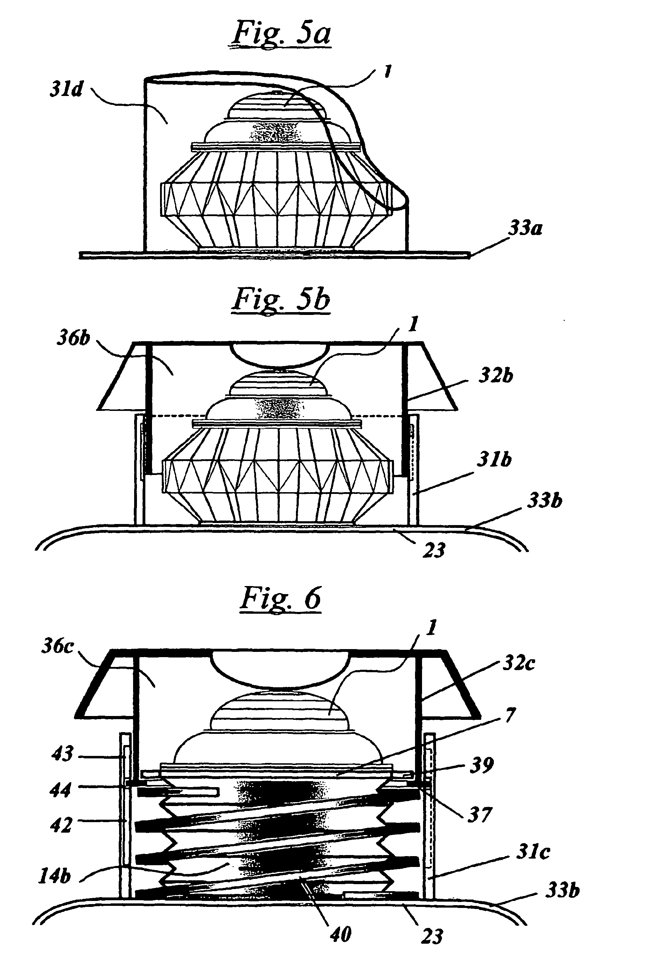

Eine Injektionseinrichtung mit Injektionsnadel in verschiedenen Betriebszuständen ist den Abbildungen 5A bis 5E sowie dem zugehörigen Teil der Beschreibung der US 5,569,190 A zu entnehmen. Eine solche Einrichtung besteht aus einem vorderen und einem darin wie ein Kolben gleitenden hinteren Gehäuse, wobei im hinteren Gehäuse ein mit einer Endkappe und einem Stößel dicht verschlossener Balg vorgesehen ist. Der Balg im hinteren Gehäuse kann entweder ein flüssiges Serum oder eine lyophilisierte Vakzine enthalten.

Für den Fall, daß der Balg im hinteren Gehäuse flüssiges Serum enthält, wird ein zusätzlicher; als Hülle oder Scheide bezeichneter, mit Flüssigkeit gefüllter Balg im vorderen Gehäuse durch eine Schraubenfeder ersetzt, weil der im vorderen Gehäuse mit starrer, zylindrischer Seitenwand vorgesehene zusätzliche Balg dann eben keine Flüssigkeit zur Auflösung einer lyophilisierten Vakzine im hinteren Gehäuse enthalten muß.

Beim Gebrauch einer solchen Einrichtung müssen allerdings erst einigermaßen umständlich zwei Sicherungen weggebrochen werden, damit das hintere in das vordere Gehäuse gleiten und sodann der Druckkolben mit Stößel in das hintere Gehäuse eindringen kann.

Die Nadel ist vor Gebrauch der Einrichtung nach der US 5,569,190 A lediglich mit einem Ende in eine Nadelaustrittsöffnung eingesteckt, welch letztere in einer Verdickung der Frontwand des vorderen Gehäuses ausgebildet ist.

Gemäß der US- A soll der Nadelaustritt allein durch das Vorrücken der Frontwand des hinteren Gehäuses bewerkstelligt werden, u. zw. ohne fixe Verankerung der rechten Nadelspitze in dieser Frontwand ("membrane"). Erst wenn ein flüssigkeitsgefüllter Balg oder eine Schraubenfeder im vorderen Gehäuse ganz zusammengedrückt ist, soll diese Frontwand - nachdem der Nadelflansch auf die Oberfläche 514 gestoßen ist - von der rechten Nadelspitze durchdrungen werden.

Schließlich soll gemäß der US- A die Nadel nach dem Zurückziehen aus der Nadelaustrittsöffnung bei erneuter Druckausübung nicht wieder durch diese Austrittsöffnung gleiten, sondern gegen die Endwand einer daran anschließenden erweiterten Öffnung stoßen.

Die Möglichkeit eines erneuten Nadelaustrittes durch die Nadelaustrittsöffnung wird jedoch offenbar nicht ausgeschlossen, denn um die Wahrscheinlichkeit eines neuerlichen Austrittes zu verringern wird vorgeschlagen, der Nadel vor dem Zusammenbau des Injektors eine geringfügige Krümmung zu verleihen. Da die Nadel aber vor Betätigung des Injektors nur durch Einstecken in die Nadelaustrittsöffnung in ihrer Lage gehalten wird, ergibt sich durch eine solche Maßnahme eine leicht gegen die Achse der Einrichtung geneigte Position der Nadel, wodurch der Nadelaustritt unter der Wirkung des Vorrückens der Frontwand des hinteren Gehäuses, in welcher die rechte Nadelspitze nicht fix verankert ist, zumindest sehr erschwert würde.

Aus der US 2,667,872 A ist eine Spritzeneinheit mit einem einstückigen, durch Blasen von Kunststoffmaterial erzeugten Medikamentenbehälter, an welchen ein Halsteil angeformt ist, bekannt. Der Halsteil weist eine mit einer Membran verschlossene Öffnung auf, in welch letzterer eine an beiden Enden zugeschärfte Injektionsnadel mittels eines kugelartigen, fest mit der Nadel verbundenen Vorsprunges eingerastet ist. Vor Gebrauch der Spritzeneinheit muß eine Nadleschutzkappe abgezogen werden und die Nadel darüber hinaus in eine zweite Raststellung bewegt werden, wobei auch die Membran durchstochen wird. Erst nach diesen vorbereitenden Manipulationen und nach Desinfektion der vorgesehenen Hautstelle kann eine Injektion verabreicht werden. Die Gefahr einer Kontamination der Nadel vor der Injektion ist entsprechend groß. Eine Verletzung durch die Nadelspitze einer gebrauchten Spritzeneinheit mit den angeführten gefährlichen Konsequenzen ist jederzeit möglich.

In der DE 14 41 377 B wird eine Spritzampulle mit einem zusammendrückbaren, becherförmigen Hohlkörper, in dem sich eine Injektionsflüssigkeit befindet, vorgeschlagen. Eine Injektionsnadel durchsetzt den gesamten Hohlkörper axial, wobei das nicht zugeschärfte, obere Nadelende in einer mit der oberen Endwandung des Hohlkörpers verbundenen Nadelplatte fixiert ist und das zugeschärfte Nadelende zunächst in einer Führung ruht, welche in einer zentralen Verdickung der unteren Endwandung des Hohlkörpers vorgesehen ist. Wird auf die obere Endwandung Druck ausgeübt, so verschieben sich Teile der abgestuft zylindrischen Seitenwand des Hohlkörpers um wenigstens einen flexiblen, ringförmigen Gelenkteil teleskopartig ineinander, das zugeschärfte Nadelende durchdringt gleichzeitig eine dünnwandige Stelle am unteren Ende der zentralen Verdickung und sticht in die Haut ein. Besonderes Augenmerk wird auf eine möglichst vollständige Entleerung des Hohlkörpers durch die Injektionsnadel sowie darauf gerichtet, dass weder Blut noch Injektionsflüssigkeit zurückgesaugt werden. Auf welchem Weg die Injektionsflüssigkeit in das obere Nadelende gelangt, ist nicht genau ersichtlich. Die Spritzampulle scheint zwar relativ einfach bedienbar, die zur Verabreichung der Injektion vorgesehene Hautstelle muß aber jedenfalls vorher desinfiziert werden. Ein Verrutschen der Spritzampulle entlang der Hautoberfläche kann nicht ausgeschlossen werden. Da die Injektionsnadel in direktem Kontakt mit der Injektionsflüssigkeit steht, könnten - speziell nach langen Lagerzeiten - zumindest geringfügige chemische Veränderungen (z. B. durch katalytische Vorgänge) in empfindlichen Injektionsflüssigkeiten ausgelöst werden, selbst wenn die Nadel beispielsweise aus korrosionsbeständigem Stahl ("Edelstahl") gefertigt sein sollte. Der gravierendste Nachteil ist schließlich darin zu sehen, dass die Nadel nach Verabreichung der Injektion aus der Spritzampulle ragt und auf diese Weise wieder ein erhebliches Gefahrenpotential durch Verletzung gegeben ist.

Die Erfindung zielt nun darauf ab, die beschriebenen Nachteile bekannter Spritzampullen zu überwinden und durch spezielle Maßnahmen eine Vielzahl von Vorteilen gegenüber den bisher vorgeschlagenen Einweg- Injektionsvorrichtungen zu erzielen.

Bei der erfindungsgemäßen Injektionsvorrichtung ist unter dem Bodenteil der Vorratskammer eine Nadelhalterung mit der Injektionsnadel vorgesehen, wobei weiters eine wenigstens einen Teil der Injektionsnadel umgebende Nadelkammer mit flexiblen Seitenwänden vorgesehen ist, und wobei in der Nadelkammer eine Einrichtung zur irreversiblen Fixierung der Injektionsnadel in der Nadelkammer nach Betätigung der Injektionsvorrichtung angeordnet ist, welche Fixiereinrichtung im wesentlichen aus einer mit einem elastischen Element zusammenwirkenden Nadelbühne besteht. Die Nadelkammer kann mit dem Bodenteil und/oder mit der Nadelhalterung verbunden sein.

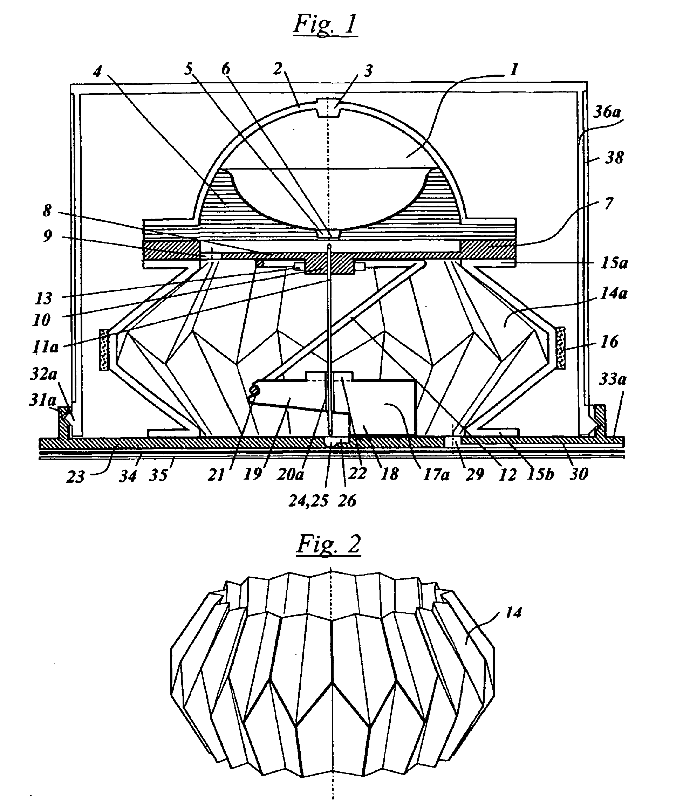

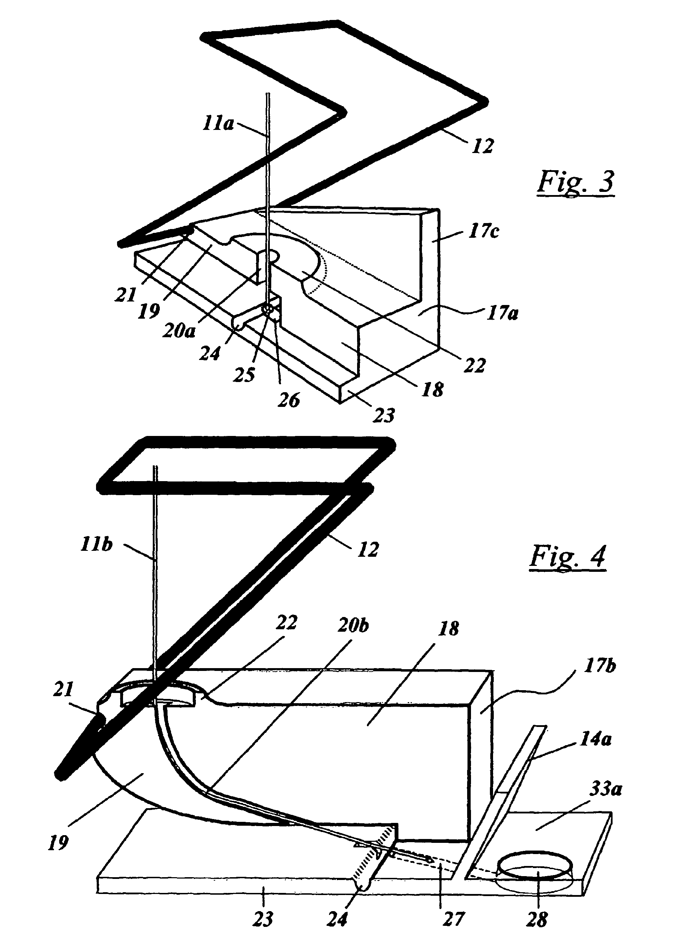

Die Seitenwände der Nadelkammer sind zweckmäßig aus einem Faltenbalg in Form eines doppelten Kegelstumpfes gebildet, wobei der Faltenbalg im Bereich seines größten Durchmessers von einem Ring aus Elastomermaterial umgeben ist. Eine solche Ausbildung der Nadelkammer ermöglicht z.B. schon mit vier Faltungen ein nahezu verwindungsfreies Niederdrücken und Strecken derselben. Der Boden der Nadelkammer setzt sich außerhalb der Seitenwand der Nadelkammer fort, um eine feste Seitenauflage der Vorrichtung auf der Hautoberfläche zu bilden und weist in einer bevorzugten Ausführungsform einen hochgezogenen Rand oder Kappensockel mit Halterungen für eine Kappe auf. Dementsprechend ist somit ein umlaufender Kappensockel für die Kappe vorgesehen. Die Halterung kann beispielsweise als kurzer Gewindegang oder nach Art eines Bajonettverschlusses gestaltet sein. Zur Vorfixierung der Vorrichtung auf der Hautoberfläche ist nach einer vorteilhaften Ausführungsform der Boden der Nadelkammer Teil eines Bandes und der hochgezogene Rand des Bodens der Nadelkammer ist zu einem hülsenförmigen Teil verlängert, in welchem eine bewegliche Kappe geführt ist. Das Band reicht um einen Körperteil, beispielsweise um einen Arm oder um ein Bein. Gemäß einer anderen bevorzugten Ausführungsform sind die Seitenwände der Nadelkammer aus einem quergefalteten, zylinderförmigen Faltenbalg gebildet Da ein solcher Faltenbalg nicht verwindungsfrei niedergedrückt werden kann, wird er in dem zu einer Hülse verlängerten Kappensockel beispielsweise mit einer Schraubenfeder als zusätzlichem Federelement geführt, welche die Kappe nach oben drückt. Erfindungsgemäß kann auch in dieser Ausführungsform die Vorfixierung auf der Hautoberfläche mittels des voranstehend erwähnten Bandes erfolgen. In beiden Fällen werden die Kappen in den Hülsen durch Führungseinrichtungen mit Quergang und Sicherung sowie verkürztem bzw. verlängertem Vertikalgang festgehalten. Die Kappen können vor Gebrauch der Vorrichtung durch Drehen - beispielsweise gegen den Uhrzeigersinn - entriegelt werden (Fig. 5, Fig. 6, Fig. 7, Fig. 8). In der Nadelkammer ist eine Nadelbühne, welche aus einem zum Boden der Nadelkammer hin (nach unten) offenen Vorderteil und einem am Boden der Nadelkammer aufstehenden Hinterteil bestehen kann, vorgesehen. Ein Teilbereich der Injektionsnadel durchsetzt vor Betätigung der Injektionsvorrichtung zweckmäßig eine Nadelführung, welch letztere vorzugsweise in der Nadelbühne integriert ist. Die Injektionsnadel erstreckt sich durch die Nadelführung, die untere Nadelspitze reicht dabei bis knapp oberhalb der Nadelöffnung im Boden der Nadelkammer (Fig. 1) oder sie ragt geringfügig in die Nadelöffnung (Fig. 4). Besonders vorteilhaft ist als Nadelführung eine zentrale Bohrung in Achsenrichtung der Nadelkammer vorgesehen. Nach einer besonderen Ausgestaltung wird der Unterteil der Injektionsnadel in einen flacheren Winkel als 90 Grad zur Hautoberfläche umgelenkt, um den Einstichwinkel nach den Anforderungen der speziellen Injektion variieren zu können. Dafür ist in der Nadelbühne eine Rille zur Umlenkung der Injektionsnadel als Nadelführung vorgesehen, wobei der obere Teil der Führungsrille in Form einer Kurve höherer Ordnung und der untere, längere Teil gerade ausgeführt ist. Die Fixiereinrichtung wird entsprechend einer Ausführungsform von einem an der Nadelhalterung befestigten Federbügel gebildet, der vor Betätigung der Injektionsvorrichtung in einer Ausnehmung der Nadelbühne eingerastet ist. Quer durch die Nadelöffnung im Bodenbereich der Nadelkammer bzw. der Nadelbühne verläuft zweckmäßig eine zweite Ausnehmurig, welche nach Betätigung der Vorrichtung den Unterteil des Federbügels aufnimmt. Der Oberteil des Federbügels kann an der Unterseite der Nadelhalterung befestigt sein. Die Nadelhalterung selbst besteht nach einer speziellen Ausgestaltung aus einer im wesentlichen kreisförmigen Platte mit wenigstens einer Öffnung und einem die Injektionsnadel unmittelbar umgebenden Anschlag. Durch diese Öffnung in der Platte ist der Druckausgleich im Inneren und die gute Sterilisierbarkeit der erfindungsgemäßen Vorrichtung gewährteistet. Die zur Spannung des Federbügels erforderliche Vertikalkraft auf die Nadelhalterung im Sinne einer Streckung der Nadelkammer wird beispielsweise durch die Spannung eines Elastomer-Ringes, der außen über den breitesten Teil einer doppelt-kegelstumpfförmigen Nadelkammer gespannt ist, bewirkt bzw. bei einer Vorrichtung mit zylindrischer, faltenbalgförmiger Wand der Nadelkammer durch das Vorsehen einer Hilfsfeder in dem zu einer Hülse hochgezogenen Kappensockel, welche Feder erst durch das Entriegeln der Kappe auf die Nadelkammer und damit auf die Nadelhalterung einwirkt. Die Nadelbühne ist nach einer zweckmäßigen Ausgestaltung mit dem Boden der Nadelkammer verbunden. Im Boden der Nadelkammer befindet sich ferner eine Druckausgleichsöffnung mit einem radial nach außen führenden, flachen Druckausgleichsgang. Wird die Vorrichtung für intrakutane Injektionen mit einer Nadelführung für flache Einstichwinkel verwendet, dann mündet die Nadelöffnung in eine beispielsweise kreisförmige Ausnehmung des Bodens der Nadelkammer, um die Ausbildung einer Quaddel der Injektionsmedien nahe der Hautoberfläche nicht zu behindern. An der Unterseite des Bodens der Nadelkammer ist eine - nach einer speziellen Ausgestaltung trägergebundene - Adhäsivschicht vorgesehen, die unterhalb der Nadelöffnung eine korrespondierende Öffnung aufweisen kann. Diese Adhäsivschicht enthält nach einer weiteren vorteilhaften Ausführungsform zusätzliche, an der Hautoberfläche oder transdermal wirksame Wirk- und Hilfsstoffe, wie beispielsweise Hautdesinfektionsmittel und schmerzstillende Stoffe. An ihrer Unterseite ist die Adhäsivschicht mit einer Schutzfolie abgedeckt, die erst knapp vor Gebrauch entfernt wird.

Die erfindungsgemäße Einweg-Injektionsvorrichtung ist gegenüber den bisher bekannten Vorrichtungen in der Anwendung
**schneller** (z.B. in der Notfallmedizin, bei anaphylaktischem Schock, bei akuter Hypoglykämie usw.),
**sicherer**. Sobald die günstigsten Einstichstellen vom Arzt festgelegt und gegebenenfalls markiert wurden, kann die Vorrichtung vom Patienten selbst angewendet werden, wobei nur minimale Einübung nötig ist.

Die Vorrichtung ermöglicht echte Einhand-Bedienung, sie ist auf vielen Körperstellen leicht aufsetzbar, das Abrutschen kann durch eine Adhäsiv(Haft)schicht verhindert werden, es ist praktisch keine Fehlbedienung möglich, vordefinierte und reproduzierbare Einstichtiefen sind erzielbar, die gesamte Vorrichtung bleibt bis zum Einstich sicher steril, ein Code bzw. ein Aufdruck verhindern Verwechslungen von Inhalt und Dosis, ein zusätzlicher Code in Braille-Schrift ermöglicht auch die Verwendung durch Blinde und Sehschwache,
**einfacher**: Kleinstmöglicher Raumbedarf der kompletten Vorrichtung, es muß ohne weitere Beachtung von Einstichwinkel oder Einstichtiefe lediglich kurzer Druck ausgeübt werden, viele Arten von Injektionsmedien können auf die gleiche Weise angewendet werden; die erfindungsgemäße Einweg-Injektionsvorrichtung ist
**auch von Patienten mit leichten Behinderungen anwendbar** (z.B. von älteren Menschen mit herabgesetztem Aufmerksamkeitsgrad), sie ist
**sogar in schwierigen motorischen Situationen** vom Patienten selbst und von Laienhelfern anwendbar (z.B. bei unkontrollierten Bewegungen wie Krampfzuständen, Tremor usw. - etwa bei akuter Hypoglykämie, dem sog. "Hypo" der Diabetiker), weiters ist eine Injektion mit einer erfindungsgemäßen Vorrichtung
**schmerzärmer** (weitgehende Schmerzfreiheit ist ein allgemeiner Vorteil von "Mikroinjektionen", zusätzlich können jedoch in der Kontaktschicht der Vorrichtung auch transdermal wirksame Anaesthetika enthalten sein), sie weist eine
**zwangsgesteuerte Abgabedynamik** auf, d. h. sowohl die Abfolge Nadeleinstich - Öffnen des Ampullenteils (der "Vorratskammer") - Wirkstoffabgabe - Zurückziehen der Nadel als auch die
Geschwindigkeit der Wirkstoffzufuhr werden allein durch Druckausübung selbsttätig geregelt, sie ist zudem **weniger belastend** für ängstliche Patienten oder Lalenhelfer (die "Nadel" und das "Einstechen" sind nicht sichtbar), die erfindungsgemäße Vorrichtung ist
**nach Gebrauch ungefährlich**, weil sie in keiner Weise wiederverwendbar ist. Die Nadelspitze wird irreversibel in die Vorrichtung zurückgezogen und dort fixiert, es entsteht kein gefährlicher Abfall, die

Vorrichtung entspricht sogar den neueren und strengeren gesetzlichen Sicherheitsbestimmungen, wie z.B. den "safety needle regulations" in den USA, sie ist ferner
**gut lager-, kühl- und transportfähig** (in Apotheken, Hausapotheken, Notfallpackungen usw.) und **kostensenkend** (z.B. hinsichtlich Personalaufwand, Infrastruktur und vermeidbaren Komplikationen).

Die Funktion der erfindungsgemäßen Vorrichtung wird unter Bezugnahme auf die in Fig.1 dargestellte Ausführungsform anhand eines subkutanen Injektionsvorgangs, z.B. der Injektion von Insulin, noch näher erläutert. Zur Kennzeichnung von Injektionsmedium und Wirkstoffmenge kann beispielsweise die Schutzfolle (35) an der Unterseite der Injektionsvorrichtung durch Beschriftung, Farbe und in erhabener Blindenschrift markiert sein. Zunächst wird die Schutzfolle (35) von der Adhäsivschicht (34) abgezogen und letztere sodann mit ihrer gesamten, die lagestabilisierende Seltenauflage (33a) einschliessenden Fläche mittels der Kappe (36a) fest auf die vorgesehene Hautoberfläche gedrückt und dadurch auf dieser fixiert. Nach kurzem Drehen der Kappe (36a) mit dem Gewinde (32a) gegen den Uhrzeigersinn kann dieselbe aus dem Kappensockel (31a) abgezogen und abgelegt werden. Enthält die Adhäsivschicht (34) als zusätzliche Wirkstoffe auch Desinfektionsmittel oder Analgetika, wartet man solange zu, bis das Eintreten der angestrebten Wirkung angenommen werden kann. Wird nun mit einem Finger, beispielsweise dem Daumen, auf den vorzugsweise aus durchsichtigem und verformbarem Material gefertigten, kuppelförmigen Oberteil (2) der mit dem fluiden, inkompressiblen Injektionsmedium gefüllten, in einer anderen Ausführungsform mit einer dem Innenraum angepaßten, das Injektionsmedium enthaltenden Kapsel gefüllten oder in einer weiteren Ausführungsform aus einer dem Innenraum angepassten und das Injektionsmedium enthaltenden Kapsel bestehenden, Vorratskammer (1) ein zunehmender Druck in Richtung senkrecht zur Hautoberfläche ausgeübt, dann erfolgt bei Überschreiten des durch das ringförmige Elastomerband (16) hervorgerufenen Gegendrucks ein gleichzeitiges Senken der Vorratskammer (1) und der Nadelhalterung (7) mit der im zylindrischen Anschlag (10) der Nadelhalterungsplatte (8) zentral fixierten, an beiden Enden zugeschärften geraden Injektionsnadel (11a). In der Nadelhalterungsplatte (8) befindet sich eine zusätzliche Arbeitsöffnung (9) für den Zusammenbau und die Sterilisation der Injektionseinrichtung. Für die an beiden Enden zugeschärfte Injektionsnadel (11a) kommen grundsätzlichen alle handelsüblichen Materialien sowie Ausbildungsformen von Nadelspitzen und Nadelschäften in Frage.

Das gemeinsame Absenken von Vorratskammer (1) und Nadelhalterung (7) wird durch das gleichzeitige, unter Vergrößerung des Umfangs des Nadelkammermittelteils erfolgende Auswölben der Wand der Nadelkammer (14a) ermöglicht Durch die besondere, in Fig. 2 genauer dargestellte Gestaltung dieser Wand in Form eines doppelten Kegelstumpfs mit Längsfaltung erfolgt der Absenkvorgang der Nadelkammer (14a) auch ohne zusätzliche Führung weitgehend verwindungsfrei. Die Wand der Nadelkammer (14a) kann mittels der abgeflachten Randstücke (15a, 15b) an die Nadelhalterung (7) und an den Boden (23) der Nadelkammer gefügt sein, oder sie geht mit ihrem unteren Rand direkt in den Boden (23). der Nadelkammer über. Die Verbindung der Wand der Nadelkammer (14a) mit der Nadelhalterung (7) und dem Boden (23) der Nadelkammer kann zweckmäßigerweise elastisch bis plastisch verformbar ausgeführt sein. Das untere Ende der nach unten bewegten Injektionsnadel (11a) tritt durch die gerade Nadelführung (20a), die Ausnehmung (24) zur Aufnahme des Federbügels (12) und die Nadelöffnung (25) im Boden der Nadelkammer in die Haut ein. Die mit dem Zusammendrücken der Nadelkammer (14a) verbundene Volumsverminderung bewirkt das Herausdrücken der Luft bzw. eines sterilen Gases aus dem Innern der Nadelkammer (14a) durch die Druckausgleichsöffnung (29) und den radial nach außen führenden. Druckausgleichsgang (30). Sobald die Injektionsnadel (11a) ausreichend tief, beispielsweise 8-10 mm, in das Gewebe eingedrungen ist und gleichzeitig der Anschlag (10) das Gegenlager (22) an der Nadelbühne (17a) berührt, bewirkt der weiter anhaltende Fingerdruck auf die Vorratskammer (1) ein Hochwölben der Nadelplatte (8) und in der Folge ein Durchstechen des Membranteils (6) im vertieften Injektionsausgang (5). Erst in diesem Moment ist durch die hohle Injektionsnadel (11a) eine offene Verbindung zwischen dem Gewebe und dem Injektionsmedium in der Vorratskammer (1) hergestellt und das Injektionsmedium kann unter dem weiter anhaltenden Fingerdruck in das Gewebe eintreten. Zugleich mit der allmählichen Entleerung der Vorratskammer (1) wölbt sich deren kuppelförmiger. Oberteil (2) nach innen und rastet schließlich beispielsweise mit dem Zapfen (3) im Injektionsausgang (5) ein, wo er festgehalten wird. Der Bodenteil (4) der Vorratskammer (1) ist konkav gewölbt, sodass der während des Entleerens nach unten gedrückte kuppelförmige Oberteil (2) schließlich gut an der Innenwand des Bodenteils (4) anliegt Auf diese Weise kommt es zu keinem Zurücksaugen des Injektionsgutes und es wird eine möglichst vollständige und gut reproduzierbare Entleerung der Vorratskammer (1) erzielt.

Das Zusammenwirken von Federbügel (12), Nadelbühne (17a). Ausnehmung (24) und Nadelöffnung (25) ist in Fig.3 genauer dargestellt Der Oberteil des Federbügels (12) ist an der Unterseite der Nadelplatte (8) fixiert, sein Unterteil in der Ausnehmung (21) der Nadelbühne (17a), die einen nach unten offenen Vorderteil (19) und einen am Boden (23) der Nadelkammer stehend befestigten Hinterteil (18) aufweist. Die beiden zueinander gebogenen Teile des Federbügels (12) stehen unter einer elastischen Spannung, durch welche der Unterteil des Federbügels nach unten gedrückt wird. Anderseits bewirkt die starke elastische Spannung des Elastomerbandes (16) der Nadelkammer (14a) unter Verminderung von deren Umfang eine vertikale Streckung derselben, wodurch der Unterteil des Federbügels in der Ausnehmung (21) insgesamt nach oben gezogen wird. Mit Beginn des Fingerdruckes auf die Vorratskammer (1) wird der Streckung der Nadelkammer (14a) entgegengewirkt, diese aufgehoben und gleichzeitig die Nadelplatte (8) mit dem Oberteil des Federbügels (12) abgesenkt, sodass dessen Unterteil aus der Ausnehmung (21) herausgeschoben wird, unter der Eigenspannung des Federbügels (12) unter den nach unten offenen Vorderteil (19) der Nadelbühne (17a) gleitet und dort auf den Boden (23) der Nadelkammer schlägt. Bis zur Berührung von Anschlag (10) und Gegenlager (22) gleitet der Federbügel auf dem Boden nach außen in Richtung der Nadelkammerseitenwand, verharrt während der Entleerung der Vorratskammer (1) in gleicher Position und gleitet bei der darauffolgenden. Aufhebung des Fingerdrucks aufgrund seiner eigenen Spannung und durch die unter der Wirkung des Elastomerbandes (16) einsetzende vertikale Streckung der Nadelkammer (14a) in umgekehrter Richtung auf die Ausnehmung (24) zu, in welcher er schließlich bleibend einrastet. Das untere Ende der injektionsnadel (11a) wird durch die vertikale Streckung der Nadelkammer (14a) wieder in dieselbe zurückgezogen. Ein abermaliges Austreten dieses Nadelendes aus der Nadelöffnung (25) ist nicht möglich, weil der in der Ausnehmung (24) bleibend eingerastete Federbügel (12) dem nochmaligen Senken der Nadelkammer unter Fingerdruck selbst einen Widerstand entgegensetzt, indem er die Nadelöffnung (25) verschließt und weil die untere Nadelspitze durch den bleibend einrastenden Federbügel zusätzlich von der Nadelöffnung (25) in Richtung einer unten geschlossenen Erweiterung (26) im Boden der Nadelkammer weggebogen wird. Selbst bei Ausübung stärkeren Druckes wird die Injektionsnadel (11a) bloß innerhalb der Nadelkammer (14a) irreversibel deformiert und aus ihrer vertikalen Lage gebracht, sodass sie nicht mehr durch die Nadelöffnung (25) austreten kann. Auch wenn die Vorratskammer (1) nur teilweise entleert und dann der Fingerdruck aufgehoben wird, tritt derselbe Mechanismus in Kraft und die spätere Weiterverwendung der Injektionsvorrichtung durch dieselbe oder eine andere Person ist nicht möglich. Zur Erleichterung des Zusammenbaues der Injektionsvorrichtung kann der Unterteil des Federbügels (12) beispielsweise über eine oben an der Nadelbühne angeformte Abschrägung (17c) in die Ausnehmung (21) gleiten.

Die folgenden Erläuterungen betreffen Beispiele weiterer Ausführungsformen der erfindungsgemäßen Vorrichtung. Fig. 4 zeigt eine erfindungsgemäße Ausführungsform der Nadelbühne für flachere Einstichwinkel als 90 Grad. So sind bei bestimmen subkutanen Injektionen beispielsweise Einstichwinkel von 45 oder 60 Grad vorteilhaft, bei intrakutanen Injektionen solche von 15 Grad. Eine Nadelführung ist hier als seitlich offene, den elastischen Schaft einer z.B. aus Edelstahl gefertigten Injektionsnadel (11 b) aufnehmende Rille (20b) in der Seitenwand der Nadelbühne (17b) ausgebildet. Bei der Fertigung kann in diese Rille eine ähnlich, aber schwächer vorgebogene Injektionsnadel (11b) seitlich eingelegt werden. Die Form der nadelführenden Rille (20b) entspricht beispielsweise im ersten Drittel einer Kurve höherer Ordnung, die sodann in ein gerades Stück übergeht. Für die Durchführung intrakutaner Injektionen unter sehr flachen Einstichwinkeln von beispielsweise 15 Winkelgraden führt die schräge Nadelöffnung (27) in die Ausnehmung (28) im Boden (23) der Nadelkammer (14a) oder in der Seitenauflage (33a), um die Bildung einer erhabenen Quaddel an der Hautoberfläche auch unter dem während des Injektionsvorgangs mit der Injektionsvorrichtung auf die Hautoberfläche ausgeübten Druck nicht zu behindern.

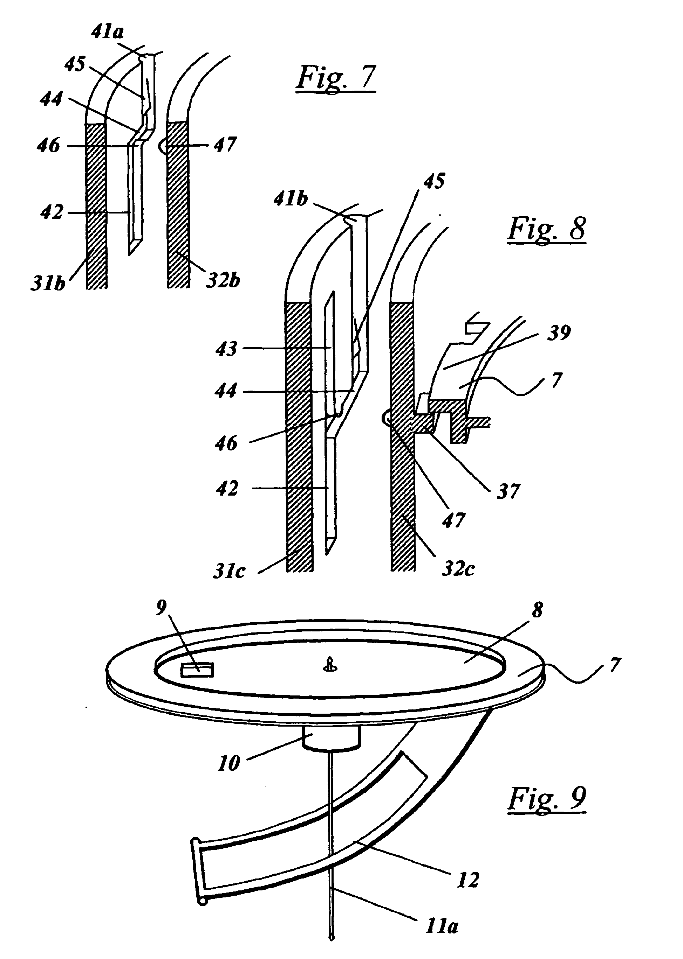

In Fig. 5 sind weitere Ausführungsformen der erfindungsgemäßen Vorrichtung dargestellt. Nach Fig. 5a ist der Kappensockel (31d) bis über die Vorratskammer (1) hochgezogen und teilweise offen ausgebildet, um ein unbeabsichtigtes Niederdrücken der Vorratskammer, beispielsweise in einer Verpackung, zu verhindern und dennoch die Injektionsvorrichtung nach der Entnahme aus der Verpackung mit einem Fingerdruck sofort auslösen zu können. In Fig. 5b ist der Boden der Nadelkammer Teil eines Bandes (33b), sodass die Injektionsvorrichtung in größerem zeitlichen Abstand von beispielsweise Stunden oder Tagen vor ihrem Gebrauch an der Körperoberfläche fixiert werden kann. In weiteren bevorzugten Ausführungsformen der erfindungsgemäßen Vorrichtung kann der Boden der Nadelkammer Teil eines Pflasters, eines Verbandes oder eines elastischen Bandes, welches Körperteile umschließt, sein. In diesen Ausführungsformen kann ferner der Kappensockel (31b) so hoch ausgeführt sein, dass er eine bewegliche Kappe (36 b) aufnehmen kann. Die an der Oberseite zur Ausübung des Fingerdrucks und zum vollständigen Niederdrücken des Oberteils (2) der Vorratskammer (1) in gleicher konkaver Rundung wie der Bodenteil (4) der Vorratskammer (1) vertiefte, bewegliche Kappe (36b) wird mit ihrer mit zumindest einer Noppe versehenen Kappenwand (32b) im Kappensockel (31b) geführt Eine für die in Fig. 5b dargestellte Ausführungsform der erfindungsgemäßen Vorrichtung vorgesehene Kappenführung ist beispielhaft in Fig. 7 wiedergegeben. Zum Einsetzen der beweglichen Kappe (36b) in den Kappensockel (31b) wird die Noppe (47) durch die Zuführung (41a) über die Kappensicherung (45) geschoben. Unmittelbar vor Gebrauch der Injektionsvorrichtung wird die Kappe gegen den Uhrzeigersinn gedreht, wodurch die Noppe (47) durch den Quergang (44) über die Auslösesicherung (46) in den Vertikalgang (42) bewegt wird. Die Injektionsvorrichtung kann nun durch Niederdrücken der beweglichen Kappe (36b) in der bereits beschriebenen Weise angewendet werden. Nach dem selbsttätigen Zurückziehen der Nadel und Verschließen der Nadelöffnung wird mit dem Band (33b) auch der Boden (23) der Nadelkammer und damit die gesamte Injektionsvorrichtung von der Körperoberfläche entfernt. Fig. 6 zeigt eine weitere bevorzugte Ausführungsform der erfindungsgemäßen Vorrichtung. In dieser ist die Nadelkammer (14b) als zylindrischer Faltenbalg mit Querfaltung gestaltet Durch die vertikale Führung der, wie bei der im Zusammenhang mit Fig. 5b beschriebenen Ausführungsform der Injektionsvorrichtung geformten, beweglichen Kappe (36c) im Kappensockel (31c) mit dem Band (33b) kann die Nadelkammer bei Ausübung des Fingerdrucks auf die bewegliche Kappe (36c) weitgehend verwindungsfrei zusammengedrückt weiden. Die für die bereits beschriebene Funktion des Federbügels (12) erforderliche vertikale Streckung der erfindungsgemäßen Nadelkammer wird in dieser Ausführungsform von einer Schraubenfeder (40) mit bewirkt Die bewegliche Kappe (36 c) wird mit ihrer mit zumindest einer Noppe versehenen Kappenwand (32c) im Kappensockel (31c) geführt Eine für die in Fig. 6 dargestellte Ausführungsform der erfindungsgemäßen Vorrichtung vorgesehene Kappenführung ist beispielhaft in Fig. 8 wiedergegeben. Zum Einsetzen der beweglichen Kappe (36c) in den Kappensockel (31c) wird die Noppe (47) durch die Zuführung (41b) über die Kappensicherung (45) geschoben. Unmittelbar vor Gebrauch der Injektionsvorrichtung wird die Kappe gegen den Uhrzeigersinn gedreht, wodurch die Noppe (47) durch den Quergang (44) über die Auslösesicherung (46) zwischen den unteren Vertikalgang (42) und den oberen Vertikalgang (43) bewegt wird und sich zugleich der Heber (37) unter den Ansatz (39) der Nadelhalterung (7) schiebt. Die Injektionsvorrichtung kann nun durch Niederdrücken der beweglichen Kappe (36c) in der bereits beschriebenen Weise angewendet werden. Dabei bewegt sich die Noppe (47) in den unteren Vertikalgang (42). Nach dem Aufheben des Fingerdruckes wird durch die Wirkung der Schraubenfeder (40) auf den Heber (37) und von diesem auf den Ansatz (39) die Nadelhalterung (7) angehoben. Dabei bewegt sich die Noppe (47) in den oberen Vertikalgang (43). Die Nädelkammer, deren Boden (23) auch die untere Begrenzung der Injektionsvorrichtung darstellt, wird beim Anheben so weit gestreckt, dass das Zurückziehen der Nadel und das Verschließen der Nadelöffnung in der bereits beschriebenen Weise bewerkstelligt wird. Abschließend wird mit der erweiterten Seitenauflage bzw. dem Band (33b) auch die gesamte Injektionsvorrichtung von der Körperoberfläche entfernt.

Fig. 9 zeigt eine weitere spezielle Ausbildung von Nadelhalterung (7) und elastischem Element (12), wobei die Nadelhalterungsplatte (8) und das elastische Element (12) in einem Stück und aus demselben dauerelastischen Material gefertigt sind.

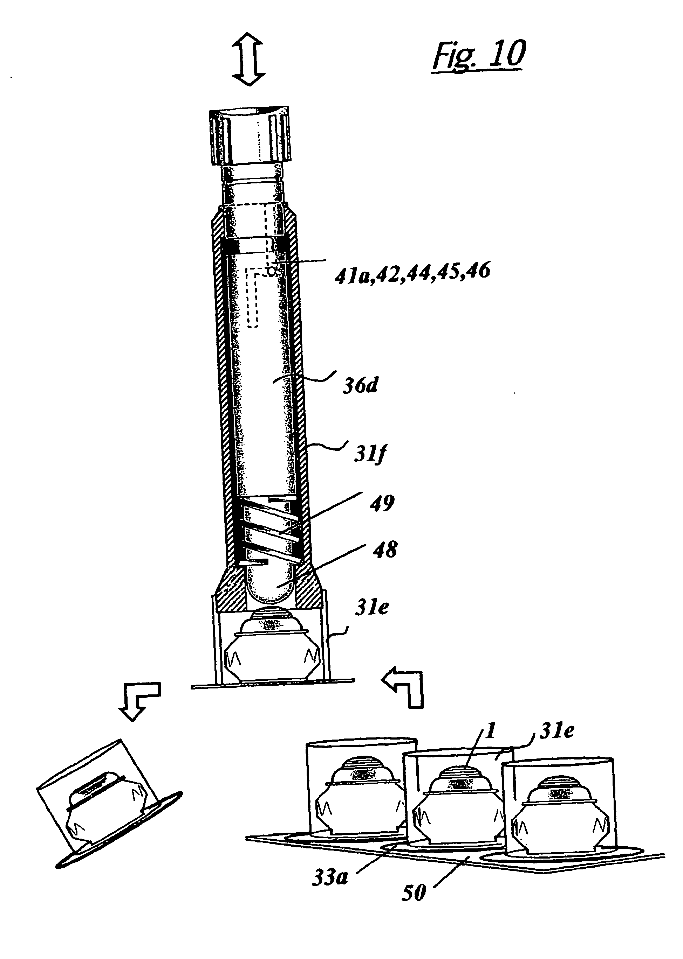

In Fig. 10 ist noch eine weitere Ausgestaltung der Injektionsvorrichtung schematisch dargestellt, wobei der Kappensockel (31e) durch ein wieder entfernbares, z.B. aufsteckbares, Zwischenstück (31f) noch weiter verlängert ist. Mehrere Vorrichtungen mit hochgezogenem Kappensockel (31e) können im Sinne von Fig. 10 auf einer gemeinsamen Trägerplatte (50) zu einer Packung vereinigt sein und diese Packung kann beispielsweise durch Einschweißen in eine transparente Kunststoff-Folie vervollständigt sein. Die bewegliche Kappe (36d) ist in diesem Falle im weiter verlängerten Kappensockel, nämlich im Zwischenstück (31f) geführt. Der Fingerdruck auf die Vorratskammer wird durch den Anpressdruck des Stößels (48), welch letzterer ein Teil der beweglichen Kappe (36d) ist, ersetzt. Die bewegliche Kappe (36d) kann, beispielsweise unter der Einwirkung eines weiteren Federelementes (49), wieder in ihre Ausgangslage rückgeführt und anschließend gemeinsam mit dem Zwischenstück (31f) nach Lösen des Kappensockels (31e) vom Zwischenstück (31f) wiederverwendet werden.

Wie bereits im Zusammenhang mit Fig. 1 genauer dargelegt, kann die Unterseite des Bodens (23) mit einer Adhäsivschicht versehen sein, welch letztere gegebenenfalls auch spezielle Hilfs- und Wirkstoffe enthält.

So kann man sich eine vorangehende Desinfektion der für eine Injektion vorgesehenen Hautstelle ersparen, wenn ein Desinfektionsmittel in der Adhäsivschicht enthalten ist. Eine Adhäsivschicht bewirkt auch die Stabilisierung der Einstichrichtung, weswegen praktisch keine unbeabsichtigten Seitwärtsbewegungen der Nadelspitze wie bei herkömmlichen Spritzen mehr möglich sind.

## Patentansprüche

1. Einweg- Injektionsvorrichtung mit einer Vorratskammer zur Aufnahme von Injektionsmedien und mit einem an der Vorratskammer vorgesehenen Injektionsausgang der von einem Ende einer an beiden Enden zugeschärften Injektionsnadel durchdringbar ist, wobei
unter dem Bodenteil (4) der Vorratskammer (1) eine Nadelhalterung (7) mit der Injektionsnadel (11a, 11b) vorgesehen ist, wobei
eine wenigstens einen Teil der Injektionsnadel (11a, 11b) umgebende Nadelkammer (14a,14b) mit flexiblen Seitenwänden vorgesehen ist, und wobei
in der Nadelkammer (14a,14b) eine Einrichtung zur irreversiblen Fixierung der Injektionsnadel in der Nadelkammer nach Betätigung der Injektionsvorrichtung angeordnet ist, welche Fixiereinrichtung im wesentlichen aus einer mit einem elastischen Element (12) zusammenwirkenden Nadelbühne (17a, 17b) besteht.

2. Einweg- Injektionsvorrichtung nach Anspruch 1, wobei ein Teilbereich der Injektionsnadel (11a, 11b) vor Betätigung der Injektionsvorrichtung eine Nadelführung (20a, 20b) durchsetzt.

3. Einweg-Injektionsvorrichtung nach Anspruch 2, wobei die Nadelführung in der Nadelbühne (17a,17b) integriert ist.

4. Einweg- Injektionsvorrichtung nach Anspruch 3, wobei in der Nadelbühne (17a) eine zentrale Bohrung (20a) in Achsenrichtung der Nadelkammer als Nadelführung vorgesehen ist.

5. Einweg-Injektionsvorrichtung nach Anspruch 3, wobei in der Nadelbühne (17b) eine Rille (20b) zur Umlenkung der Injektionsnadel (11b) als Nadelführung vorgesehen ist.

6. Einweg- Injektionsvorrichtung nach den Ansprüchen 3 bis 5, wobei die Nadelbühne (17a, 17b) mit dem Boden (23) der Nadelkammer (14a,14b) verbunden ist.

7. Einweg- Injektionsvorrichtung nach den Ansprüchen 1 bis 6, wobei der Boden (23) der Nadelkammer einen hochgezogenen Rand oder Kappensockel (31a) mit Halterungen (32a) für eine Kappe (36a) aufweist.

8. Einweg- Injektionsvorrichtung nach Anspruch 7, wobei der Boden (23) der Nadelkammer (14a, 14b) Teil eines Bandes (33b) ist und der hochgezogene Rand des Bodens der Nadelkammer zu einem hülsenförmigen Teil (31b, 31c) verlängert ist, in welchem eine bewegliche Kappe (36b, 36c) geführt ist.

9. Einweg- Injektionsvorrichtung nach den Ansprüchen 1 bis 8, wobei die Seitenwände der Nadelkammer (14a) aus einem Faltenbalg in Form eines doppelten Kegelstumpfes gebildet sind, wobei der Faltenbalg im Bereich seines größten Durchmessers von einem Ring (16) aus Elastomermaterial umgeben ist.

10. Einweg- Injektionsvorrichtung nach den Ansprüchen 1 bis 8, wobei die Seitenwände der Nadelkammer (14b) aus einem zylindrischen Faltenbalg gebildet sind und ein zusätzliches Federelement (40) vorgesehen ist.

11. Einweg- Injektionsvorrichtung nach den Ansprüchen 1 bis 10, wobei die Nadelhalterung (7) aus .einer im wesentlichen kreisförmigen Platte (8) mit wenigstens einer Öffnung (9) und einem die Injektionsnadel (11a, 11b) unmittelbar umgebenden Anschlag (10) besteht.

12. Einweg- Injektionsvorrichtung nach den Ansprüchen 1 bis 11, wobei die Fixiereinrichtung von einem an der Nadelhalterung (7) befestigten Federbügel (12) gebildet wird, der vor Betätigung der Injektionsvorrichtung in einer Ausnehmung (21) der Nadelbühne (17a, 17b) eingerastet ist.

13. Einweg-Injektionsvorrichttung nach Anspruch 12, wobei in der Nadelbühne (17a, 17b) eine zweite Ausnehmung (24) zur Aufnahme des Federbügels (12) nach Betätigung der Injektionsvorrichtung vorgesehen ist.

14. Einweg- Injektionsvorrichtung nach den Ansprüchen 1 bis 13, wobei an der Unterseite des Bodens (23) der Nadelkammer (14a, 14b) eine Adhäsivschicht (34) vorgesehen ist, welch letztere gegebenenfalls zusätzliche, an der Hautoberfläche oder transdermal wirksame Wirk- und Hilfsstoffe wie Desinfektionsmittel und Analgetika enthält.

## Claims

1. A disposable injection device comprising a reservoir chamber to contain injection media having an injection exit, that may be penetrated by one end of a double-pointed injection needle;
a needle holder (7) integral with the injection needle (11a, 11b) below the bottom (4) of the reservoir chamber (1);
a needle chamber (14a, 14b) with flexible walls that covers the injection needle (11 a, 11 b) at least partially;
an equipment inside the needle chamber (14a, 14b) to irreversibly fix the injection needle inside the needle chamber after operation of the injection device, with the fixation equipment basically consisting of an elastic element (12) working together with a needle stage (17a, 17b).

2. Disposable injection device of claim 1, wherein a part of the injection needle (11a, 11b) is situated in a needle duct (20a, 20b) before operation.

3. Disposable injection device of claim 2, wherein the needle-duct is integrated into the needle stage (17a, 17b).

4. Disposable injection device of claim 3, wherein the needle stage (17a) has a central bore (20a) in the needle chamber's axial direction to serve as a needle-duct.

5. Disposable injection device of claim 3, having a grooved guiding path (20b) in the needle stage (17b) to turn round the injection needle (11b), thus serving as a needle duct.

6. Disposable injection device of claims 3 to 5, wherein the needle stage (17a, 17b) is connected to the bottom (23) of the needle chamber (14a, 14b).

7. Disposable injection device of claims 1 to 6, wherein the bottom (23) of the needle chamber bears an upwards-extended brim or cap base (31 a) with a cap (36a) holder (32a).

8. Disposable injection device of claim 7, wherein the bottom (23) of the needle chamber (14a, 14b) is part of a band (33b), and the upwards-extended brim of the needle chamber's bottom (23) is lengthened sleeve-like (31b, 31c), containing a movable guided cap (36b, 36c).

9. Disposable injection device of claims 1 to 8, wherein the needle chamber's (14a) sidewalls are built of a bellow in the form of a double frustrum of a cone, and a ring (16) of elastomere material girdles the bellow at the level of its largest diameter.

10. Disposable injection device of claims 1 to 8, wherein the walls of the needle chamber (14b) are built of a cylindric bellow, and an additional spring element (40) is provided

11. Disposable injection device of claims 1 to 10, wherein the needle holder (7) consists of a basically circular plate (8) with at least one opening (9), and a block unit (10) that encloses the injection needle (11a, 11b).

12. Disposable injection device of claims 1 to 11, wherein the fixation equipment consists of a spring clip (12) that is fixed to the needle holder (7) and that is caught in a notch (21) in the needle stage (17a, 17b) before operation.

13. Disposable injection device of claim 12, having a second notch (24) in the needle stage (17a, 17b) to take up the spring clip (12) after operation of the injection device.

14. Disposable injection device of claims 1 to 13, having an adhesive layer (34) on the lower side of the needle chamber's (14a, 14b) bottom (23), wherein the adhesive layer may contain additional active substances and adjuvants, like for example disinfectants or analgetics, that act trandermally or take effect directly on the skin.

## Revendications

1. Dispositif d'injection jetable avec une réserve pour la réception du média d'injection sur la réserve qui est sur sa sortie transperceable par l'aiguille d'injection pointue aux deux bouts celui ci comprenant sous le sol (4) de la réserve (1) une fixation pour aiguille (7) avec aiguille d'injection (11a,11b) ainsi que au minimum une partie de l'aiguille d'injection (11a, 11b) entourée d'une chambre d'aiguille (14a, 14b) avec parois flexible ainsi que la chambre d'aiguille (14a, 14b) comporte un Dispositif pour la fixation irréversible de l'aiguille d'injection dans la chambre d'aiguille après l'utilisation, il est également à noter se trouve être le Dispositif de fixation qui se compose en principal d'un élément élastique (12) agissant avec un socle d'aiguille (17a,17b).

2. Dispositif d'injection jetable selon la revendication 1 avec une partie de l'aiguille d'injection (11a,11b) transperce un conduit d'aiguille (20a, 20b) avant l'utilisation du Dispositif d'injection.

3. Dispositif d'injection jetable selon la revendication 2 avec conduite d'aiguille intégrée dans le socle d'aiguille (17a, 17b).

4. Dispositif d'injection jetable selon la revendication 3 avec ouverture centrale (20a) dans l'axe de la chambre d'aiguille pour la conduite de l'aiguille située dans le socle d'aiguille (17a).

5. Dispositif d'injection jetable selon la revendication 3 avec une fente de conduite (20b) qui est pour la déviation de l'aiguille d'injection (11 b) inclus dans le socle d'aiguille.

6. Dispositif d'injection jetable selon l'une des revendications 3 à 5 avec le socle d'aiguille (17a,17b) fixé avec le sol (23) de la chambre d'aiguille (14a,14b).

7. Dispositif d'injection jetable selon l'une des revendications 1 à 6 avec bord relevé ou rebord pour couvercle formé par le sol (23) de la chambre d'aiguille avec fixations (32a) pour couvercle (36a).

8. Dispositif d'injection jetable selon la revendication 7 avec le sol (23) de la chambre d'aiguille (14a,14b) faisant parti d'une bande (33b) ainsi que le bord relevé du sol de la chambre d'aiguille étant rallongé en une pièce formant un étui (31b, 31c) dans lequel est conduit un couvercle amovible (36b, 36c)

9. Dispositif d'injection jetable selon l'une des revendications 1 à 8 avec les parois de la chambre d'aiguille (14a) étant formées d'une forme pliable en accordéon ayant la forme d'une pyramide conique double entourée dans son plus gros diamètre d'un anneau (16) en matière élastomère

10. Dispositif d'injection jetable selon l'une des revendications 1 à 8 avec les parois de la chambre d'aiguille (14b) formées d'un cylindre pliable en accordéon et pourvu d'un élément d'amorti à ressort supplémentaire (40).

11. Dispositif d'injection jetable selon l'une des revendications 1 à 10 avec la fixation d'aiguille (7) étant formée d'une plaque principalement cylindrique (8) avec au minimum une ouverture (9) et une aiguille d'injection (11a, 11b) entourée de la base (10).

12. Dispositif d'injection jetable selon l'une des revendications 1 à 11 avec Dispositif de fixation étant constitué d'un ressort à arc (12) fixé à la fixation d'aiguille (7) qui sera encastré avant l' utilisation du Dispositif d'injection dans un renfoncement (21) du socle d'aiguille (17a,17b).

13. Dispositif d'injection jetable selon la revendication 12 avec dans le socle d'aiguille (17a, 17b) un second renfoncement (24) pour la réception du ressort à arc (12) après utilisation du Dispositif d'injection.

14. Dispositif d'injection jetable selon l'une des revendications 1 à 13 avec la partie inférieure du sol (23) de la chambre d'aiguille (14a,14b) pourvue d'une couche adhésive (34) qui transmet si nécessaire à la surface de la peau ou de façon transdermale des substances d' aide, de désinfection ou d'analgetique.
